# EUROPEAN PATENT APPLICATION

(11) **EP 4 331 499 A1**
(43) Date of publication of application: **06.03.2024**
(21) Application number: 22192563.9
(22) Date of filing: 29.08.2022
(51) Int. Cl.: A61B 8/08

(54) **ULTRASOUND IMAGING SYSTEMS AND METHODS**

(71) Applicant: Koninklijke Philips N.V., 5656 AG Eindhoven (NL)
(72) Inventor: LORENZ, Cristian, Eindhoven (NL); ORASANU, Eliza Teodora, Eindhoven (NL); SCHMIDT-RICHBERG, Alexander, 5656AG Eindhoven (NL); FRANZ, Astrid Ruth, 5656AG Eindhoven (NL)
(74) Representative: Philips Intellectual Property & Standards

(57) **Abstract**

According to an aspect, there is provided a computer implemented method performed as part of an ultrasound, US, examination of a subject. The method comprising: i) causing a three-dimensional, 3D, ultrasound probe to obtain a first 2D US image, wherein the first 2D US image is obtained in a first scanning plane of the probe; ii) causing the US probe to obtain one or more other 2D US images in a sub-volume surrounding the first scanning plane, wherein the sub-volume has a smaller spanning angle than that of a 3D mode of the probe; and iii) determining whether a target is closer to the first 2D US image or one of the one or more other 2D US images.

## Description

### FIELD OF THE INVENTION

The disclosure herein relates to ultrasound imaging. More specifically, but non-exclusively, the disclosure relates to providing directional guidance to image a target through a subject during an ultrasound examination.

### BACKGROUND OF THE INVENTION

Ultrasound (US) is the modality of choice for many examinations, such as fetal screening, as it is able to render the anatomy in sufficient detail, while at the same being time and cost effective with no known adverse effects.

The screening procedure is to some extent standardized by the International Society of Ultrasound in Obstetrics and Gynecology (ISUOG), but specific variations exist in different geographies. Generally, fetal screening is performed in specific standardized fetal anatomical views in order to obtain appropriate and standardised growth measures. Some of these standardised views are described in the paper by: A. T. Papageorghiou, et al.: entitled: "International standards for fetal growth based on serial ultrasound measurements: The Fetal Growth Longitudinal Study of the INTERGROWTH-21st Project"; The Lancet 384, 869-879, 2014.

Other US examinations are similarly standardised and require measurements and/or assessment of anatomical features in particular view planes. Examples include but are not limited to, brain scans, liver scans and heart scans.

To find and assess the appropriate standard planes in an US examination, many anatomical views need to be quickly navigated assessed.

Worldwide there is a large variety in obstetrical expertise of the people performing fetal and other types of screenings. In the US for example, a general practitioner may perform screenings, e.g. rather than a practitioner with specialist ultrasound or obstetrical expertise. This can be inefficient, particularly if it takes the practitioner a long time to find the appropriate standardised planes and/or can be inaccurate if an incorrect plane is inadvertently imaged.

### SUMMARY OF THE INVENTION

Due to the large number of anatomical views that need to be investigated during US examinations such as fetal scans, screening quality in terms of completeness and accuracy can be low. Due to the inherent difficulties of ultrasound and the challenge of examining a patient within a patient, the less skilled practitioner may have difficulty navigating the ultrasound probe in order to acquire the correct view for a given biometrical measurement or organ assessment. It is an object of embodiments herein to improve this situation.

According to a first aspect, there is provided a computer implemented method performed as part of an ultrasound, US, examination of a subject. The method comprising: i) causing a three-dimensional, 3D, ultrasound probe to obtain a first 2D US image, wherein the first 2D US image is obtained in a first scanning plane of the probe; ii) causing the probe to obtain one or more other 2D US images in a sub-volume surrounding the first scanning plane, wherein the sub-volume has a smaller spanning angle than that of a 3D mode of the probe; and iii) determining whether a target is closer to the first 2D US image or one of the one or more other 2D US images.

According to a second aspect, there is provided an apparatus for performing an ultrasound, US, examination of a subject. The apparatus comprises: a memory comprising instruction data representing a set of instructions; and a processor configured to communicate with the memory and to execute the set of instructions, wherein the set of instructions, when executed by the processor, cause the processor to: i) obtain a first 2D US image using a three-dimensional, 3D, ultrasound imaging probe, wherein the first 2D US image is obtained in a first scanning plane of the probe; ii) obtain one or more other 2D US images in a sub-volume surrounding the first scanning plane, wherein the sub-volume has a smaller spanning angle than that of a 3D mode of the probe; iii) determine whether a target is closer to the first 2D US image or one of the one or more other 2D US images.

According to a third aspect, there is an ultrasound, US, imaging system comprising a three-dimensional, 3D, ultrasound probe. The US imaging system is configured to cause the probe to operate in a scanning mode whereby the probe: v) obtains a first 2D US image in a first scanning plane of the probe; vi) obtains one or more other 2D US images in a sub-volume surrounding the first scanning plane, wherein the sub-volume has a smaller spanning angle than that of a 3D mode of the probe; and repeats steps v) and vi) in a continuous manner.

Thus, in embodiments herein, a 3D probe is used in a 2D acquisition mode to obtain a first 2D IS image and one or more other 2D US images in a surrounding sub-volume. The first image and the one or more other images are then processed to determine whether a target, such as a target structure or target imaging plane, is closer to the first 2D US image, or one of the one or more other 2D US images. This may be used to provide directional guidance to a user or operator of the US probe in order to guide the user to obtain an image of the target.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 is an example apparatus according to some embodiments herein;
Fig. 2 shows an example Ultrasound Imaging System according to some embodiments herein;
Fig. 3 shows an example method according to some embodiments herein;
Fig. 4 shows an US probe and example imaging planes of an example probe.
Fig. 5 shows an example output of a YOLO network according to some embodiments herein;
Fig. 6 shows example directional guidance according to some embodiments herein; and
Fig. 7 shows an example GUI workflow visualiser.

### DETAILED DESCRIPTION OF EMBODIMENTS

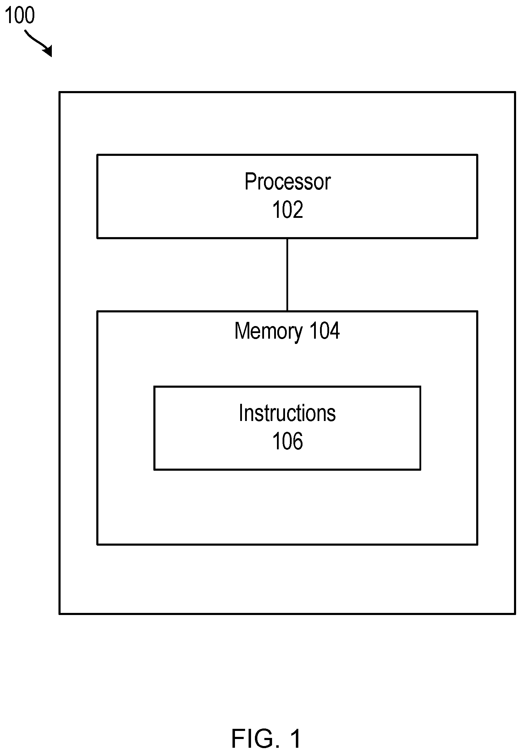

Turning now to Fig. 1 in some embodiments there is an apparatus 100 for performing an ultrasound, US, examination of a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 300 as described below.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the first 2D US image, the one or more other 2D US images and/or any other information or data received, calculated or determined by the processor 102 of the apparatus 100 or from any interfaces, memories or devices that are external to the apparatus 100. The processor 102 may be configured to control the memory 104 to the first 2D US image, the one or more other 2D US images and/or the any other information or data.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

Generally, embodiments of the apparatus 100 may be for use in performing an US examination of a subject. For example, some embodiments of the apparatus 100 may be for providing directional guidance to image a target through a subject during an ultrasound, US, examination. More specifically, the set of instructions, when executed by the processor, cause the processor to: i) obtain a first 2D US image using a three-dimensional, 3D, ultrasound imaging probe, wherein the first 2D US image is obtained in a first scanning plane of the probe; ii) obtain one or more other 2D US images in a sub-volume surrounding the first scanning plane, wherein the sub-volume has a smaller spanning angle than that of a 3D mode of the probe; and iii) determine whether a target is closer to the first 2D US image or one of the one or more other 2D US images.

In some embodiments there is an ultrasound, US, imaging system comprising a three-dimensional, 3D, ultrasound probe. The US imaging system is configured to cause the probe to operate in a scanning mode whereby the probe: v) obtains a first 2D US image in a first scanning plane of the probe; vi) obtains one or more other 2D US images in a sub-volume surrounding the first scanning plane, wherein the sub-volume has a smaller spanning angle than that of a 3D mode of the probe; and repeats steps v) and vi) in a continuous manner.

For example, in the scanning mode herein, the 3D probe is configured to obtain the first 2D US image, and the one or more other 2D US images in a continuous loop, without obtaining a (full) 3D image. In other words, without obtaining images in the full 3D volume available to the 3D probe.

In some embodiments, the apparatus 100 is part of an US imaging system. For example, an US imaging system may comprise the apparatus 100, an imaging probe and a display to display the first 2D US image. As described in more detail below, in some embodiments, the display may further display directional guidance, the directional guidance comprising a direction in which to move the US probe to image the target, based on the results of step iii).

The apparatus may be configured to instruct or cause the probe to perform steps v) and vi). E.g. the apparatus may be configured to cause the probe to operate in the scanning mode described above. In some embodiments, the apparatus 100 may thus obtain the first 2D US image by instructing or causing the probe to obtain the first 2D US image in the first scanning plane. The apparatus 100 may further obtain the one or more other 2D US images by instructing or causing the probe to obtain the one or more other 2D US images.

In some embodiments, only the first 2D US image is displayed on the display, and the one or more other 2D US images are (only) for use in determining the directional guidance. The ultrasound imaging system may be configured to repeat steps i), ii), and iii) displaying only the first image to the user in each iteration. In other words the one or more other 2D US images in the sub-volume may be processed at the same time as the first 2D US image (e.g. as a background process), in order to determine directional guidance.

In other words, the US image acquisition process may alternate between obtaining a first 2D US image in the first scanning plane and one or more other 2D US images in a sub-volume surrounding the first scanning plane. Only the first 2D US image in each iteration may be displayed to the user. The other 2D US images in each iteration may be used by the US image system to determine e.g. directional guidance. Put another way, the other 2D US images may be obtained without the knowledge of the sonographer performing the US examination, for example, the sonographer may move the US probe in order to obtain the first 2D US image, and the probe may be configured (or instructed) to obtain the other 2D US images in an automated manner after the first 2D US image, in order to determine the directional guidance for the user. In this manner, a "2.5"D mode may be achieved, where the user is working in a 2D imaging mode, while in the background, the US imager is obtaining other 2D US images in different planes to the primary imaging plane being used by the user, that can be processed in order to provide directional guidance to the user. If the Field of view of the sub-volume is small enough, then there is the possibility of realizing the '2.5D' mode, e.g. acquiring side frames in addition to the central frame, without compromising the 2D mode.

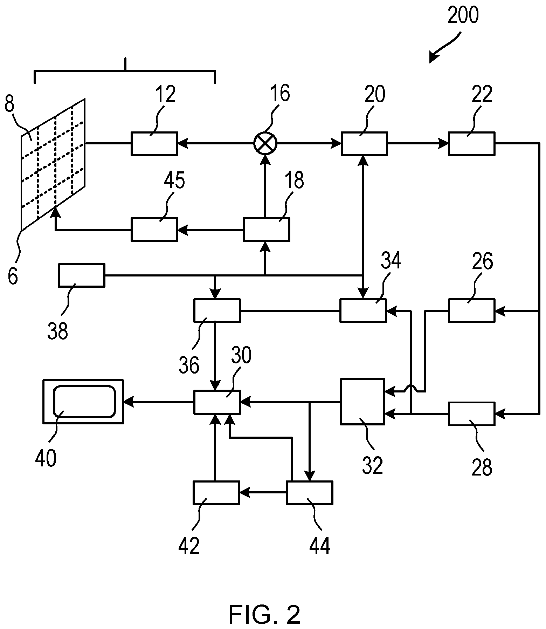

An example US imaging system 200 is shown in Fig. 2. An US imaging system may further comprise other components, such as those associated with obtaining and processing US image data. US system 200 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 2).

The system 200 may further comprise a transmit/receive (T/R) switch 16, which the microbeamformer 12 can be coupled to and which switches the array between transmission and reception modes. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

It will be appreciated that the US image system illustrated in Fig 2 is merely an example and that an US image system may comprise different components to those described above.

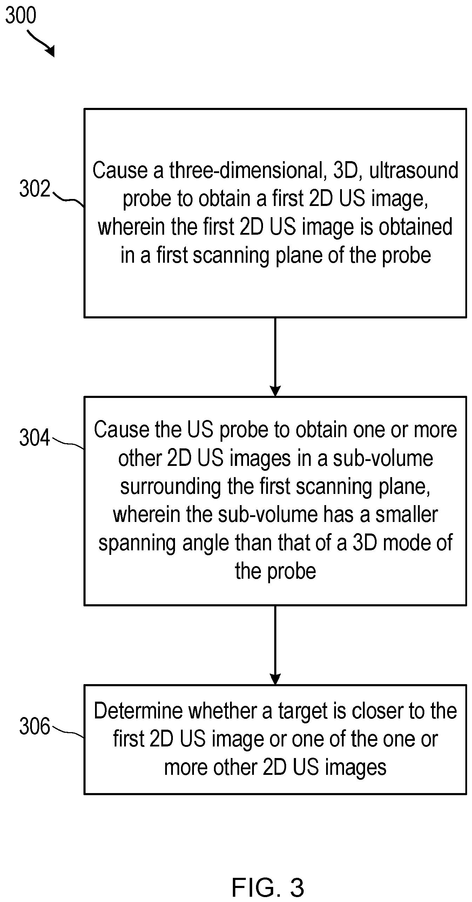

Turning to Fig. 3, there is a computer implemented method 300 that can be performed as part of an US examination of a subject. Embodiments of the method 300 may be used to provide directional guidance to a user (e.g. sonographer) to image a target through a subject during an ultrasound, US, examination.

Briefly, in a first step 302, the method 300 comprises: i) causing a three-dimensional, 3D, ultrasound probe to obtain a first 2D US image, wherein the first 2D US image is obtained in a first scanning plane of the probe. In a second step 304, the method 300 comprises: ii) causing the US probe to obtain one or more other 2D US images in a sub-volume surrounding the first scanning plane, wherein the sub-volume has a smaller spanning angle than that of a 3D mode of the probe. In a third step 306, the method 300 comprises: iii) determining whether a target is closer to the first 2D US image or one of the one or more other 2D US images.

In some embodiments, the method may be performed by a computing apparatus such as the apparatus 100. The method 300 may be performed by an ultrasound, imaging system, such as the US imaging system 200 described above. It will also be appreciated that the metho 300 may be performed by a computing system remote to an US imaging system (e.g in the cloud), for example an US imaging system may communicate with a remote computing system by sending the first 2D US images and the one or more other 2D US images to the remote imaging system for process. The remote computing system may send one or more outputs (such as the directional guidance) back to the US imaging system.

The method 300 may be for use in imaging a target through a subject during an US examination. The subject may be a human subject, for example, fetus, a child or adult. The subject may be an animal subject.

The US examination may be, for example, a fetal examination, e.g. a fetal growth scan or a fetal anomaly scan. In other embodiments, the US examination may be a heart scan, a brain scan, or any other type of US examination. Generally, the US examination may be any type of examination in which the sonographer (e.g. clinical user of an ultrasound system) is trying to image a particular plane or slice through the subject.

The target may be input by a user. For example, the method 300 may further comprise receiving a user input indicating the target. A user input may be received, for example, from a user input device such as a keyboard, mouse, or any other user input device. Alternatively, the target may be obtained from a database or other clinical system. For example, e.g. from a database, medical guideline, or medical schema.

The target may be a target structure in the subject (e.g. a target anatomical structure). For example, the target may be the head, the leg, the abdomen, the heart, or any other organ or body part of a fetus. The method 300 may be used to direct the user of the probe to obtain an image of the target. For example the method 300 may be used to direct the user of the probe from imaging a current anatomical feature to the target structure (e.g. from the fetus' abdomen to head, for example),

In some embodiments the target may be a target plane through the subject. A target plane may be a particular plane, e.g. a plane of interest through the subject. In some embodiments, the target plane may be a standardised imaging plane. For example, an imaging plane in which a particular measurement is to be made according to a medical guideline or medical standard.

Examples of target planes include but are not limited to the Trans-thalamic brain plane, the Abdominal circumference plane and the Femur biometry plane which are standardised planes used in Fetal Examinations.

A target plane may be associated with (e.g. defined by) a first plurality of anatomical features. In other words, the first plurality of features may need to be present in an image in order for the image to have correctly captured the target plane. Conversely, a target plane may be defined by the absence of a second plurality of anatomical features. In other words, the second plurality of features may need to be absent from an image in order for the image to have correctly captured the target plane.

Some example standardised planes (that may be the target plane in an US examination) are given in column 1 of Table 1 in Appendix I. Example first pluralities of anatomical features for each respective standardised plane (e.g. anatomical features that should be present in order for the standardised plane to have been correctly imaged) are given in the second and third columns of Table 1. Example second pluralities of anatomical features (e.g. anatomical features that should not be present in order for the standardised plane to have been correctly imaged) are given in the fourth column of Appendix 1. Further examples of standardised planes are described in the paper by A. T. Papageorghiou, et al. (2003) referenced in the Background section of this document.

As noted above, in some embodiments, the method 300 can be used to provide directional guidance that can be used by a sonographer (or any other user) in order to help the sonographer to move an US probe in order to image the target plane through the subject.

In step i) a 3D US probe is caused to obtain 302 a first 2D US image in a first scanning plane of the probe. For example, step i) may comprise sending a message to the probe to instruct the probe to obtain the first 2D US image. In other words, a 3D probe is used to obtain the first 2D image. The first 2D US image is obtained in a first scanning plane of the probe.

The first scanning plane generally corresponds to the plane viewed by the sonographer (e.g. displayed by a display associated with the US imaging system described above) while performing the US examination. The first scanning plane of the probe may thus correspond to a "default" plane scanned by the 3D probe when the 3D probe is used in a 2D mode. For example, a primary plane of the probe.

The primary plane may correspond to a plane that is central, or in the middle of all, of the possible planes that the 3D probe may image whilst in a 3D mode. Thus, the purpose of the method 300 may be to capture the target in the first scanning plane of the probe (e.g. that is being viewed by the sonographer).

The first 2D US image may be obtained in real-time from the 3D probe, e.g. as part of a sequence of ultrasound images (or frames) received from the probe.

In step ii) the US probe is caused to obtain one or more other 2D US images are obtained 302 in a sub-volume surrounding the first scanning plane. For example, step ii) may comprise sending a message to the probe to instruct the probe to obtain the one or more other 2D US images. In other words, the one or more other 2D US images may be obtained in a sub-portion of the 3D field of view (e.g. the portion around the central 2D view), instead of normal 3D operating mode (i.e. scanning the whole field of view). The sub-volume has a smaller spanning angle than that of the full 3D mode.

The one or more other 2D US images may be obtained by means of a wobble about the central scanning plane of the probe.

Obtaining the one or more other 2D images in a sub-volume of the full 3D volume allows for a higher frame rate than scanning the whole 3D volume. This allows US images to be obtained along the first scanning plane and displayed to the user of the probe in a real-time manner, while also obtaining the one or more other images for (internal) analysis and guidance purposes without the user being aware of a delay.

As an example, in some embodiments, the sub-volume may be a volume adjacent to and/or either side of the first scanning plane. In some embodiments, in step ii), the one or more other 2D US images may be in scanning planes adjacent to the first scanning plane. For example, the one or more other 2D images may comprise second and/or third 2D US images in second and/or third scanning planes of the probe. The second and/or third scanning planes are different planes to the first scanning plane of the probe. The second and/or third scanning planes may be adjacent to the first scanning plane (e.g. next to the first scanning plane). For example, the second and third scanning planes may lie either side of the first scanning plane. The second and third scanning planes may be adjacent to the first scanning plane.

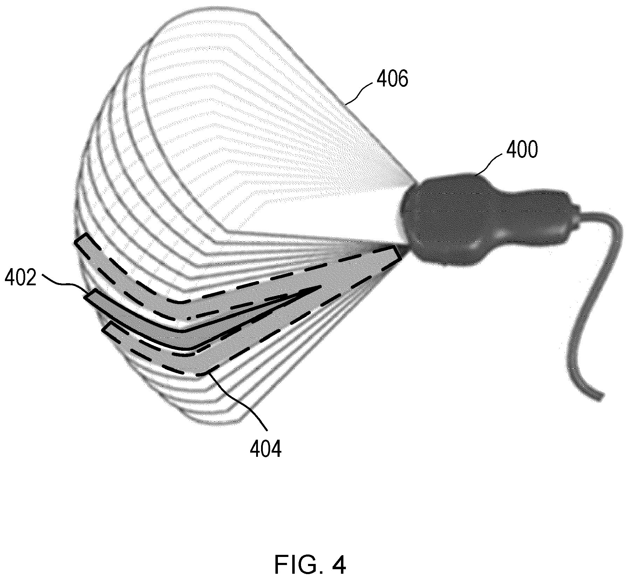

This is illustrated in Fig. 4 which shows an example according to some embodiments herein. In this embodiment, there is a first scanning plane 402, a second scanning plane 404 and a third scanning plane 406 of a probe 400. The first scanning plane 402 is along the central axis of the probe that is used as the default axis when using the probe in its 2D mode. The probe 400 sends a first 2D US image obtained in the first scanning plane 402 to an US imaging system for display on a display associated with the US imaging system. The probe 400 also obtains second and third 2D US images along the second and third planes 402, 404 for use in step iii), e.g. for use in determining whether a target plane is closer to the first, second or third scanning planes. Although three 2D US images are illustrated in Fig. 4, it will be appreciated that this is merely an example and that the one or more other 2D US images may comprise any number of 2D US images in a sub-volume surrounding the first image plane. Furthermore, the placing of the 2D US images in Fig. 4 is also merely an example, and the one or more other 2D US images may alternatively all be on the same side of the first image plane, or in any other configuration.

In step iii) the method 300 comprises determining whether the target is closer to the first 2D US image or to one of the other 2D US images. Closer, as used herein, may refer to distance, or for example, closeness in terms of feature correspondence. For example, a 2D US image may be closer to a target plane if it captures a higher proportion of the anatomical features associated with the target plane, compared to another 2D US image that captures a lower proportion of the anatomical features associated with the target plane.

In some embodiments, the closeness of each 2D US image may be expressed in terms of a score, ranking or other rating.

In some embodiments, step 306, the step of determining, comprises using a machine learning model to determine a first confidence score describing how closely the first 2D US image corresponds to the target plane and a second confidence score describing how closely the one of the one or more other 2D US images corresponds to the target plane.

The skilled person will be familiar with machine learning (ML) and machine learning processes for use in training machine learning models. ML models may be trained in a supervised manner, for example, using a training data set comprising training examples (each training example comprising an example input and a corresponding "correct" ground truth output). The model is trained on the training data, using a machine learning process.

In such embodiments, the confidence scores may be an aggregation (e.g. summation, mean or median) of a plurality of confidence scores, each of the plurality of confidence scores reflecting a confidence with which the machine learning model detects a respective anatomical feature associated with the target, in the respective image.

A machine learning process comprises a procedure that is run on the training data to create the machine learning model. The machine learning process comprises procedures and/or instructions through which training data, may be processed or used in a training process to generate the machine learning model. The machine learning process learns from the training data. For example, the process may be used to determine how one set of parameters in the training data (input parameters of the model) are correlated with another set of parameters in the training data (output parameters of the model). The machine learning process may be used to fit the model to the training data.

The model, or machine learning model, may comprise both data and procedures for how to use the data to e.g. make the predictions described herein. The model is the result (e.g. output) of the machine learning (e.g. training) process, e.g. a collection of rules or data processing steps that can be performed on the input data in order to produce the output. As such, the model may comprise e.g. rules, numbers, and any other algorithm-specific data structures or architecture required to e.g. make predictions.

Different types of models take different forms. Some examples of machine learning processes and models that may be used herein include, but are not limited to: linear regression processes that produce models comprising a vector of coefficients (data) the values of which are learnt through training; decision tree processes that produce models comprising trees of if/then statements (e.g. rules) comprising learnt values; and neural network models comprising a graph structure with vectors or matrices of weights and biases with specific values, the values of which are learnt using machine learning processes such as backpropagation and gradient descent.

In some embodiments herein, a You Only Look Once (YOLO) network described in the paper by Redmon et al. (2016) entitled "You Only Look Once: Unified, Real-Time Object Detection*"* may be trained to detect the target in US images in real-time in US image feeds.

As another example, a U-net network described in paper "U-Net: Convolutional Networks for Biomedical Image Segmentation" by Olaf Ronneberger et al. can be trained to segment out features corresponding to the target US image feeds.

The skilled person will appreciate however that these are merely examples, and that there are many ways that a target may be detected in US images and US image feeds, for example, using techniques such as Radial Gradient Index (RGI) Filtering, Multifractal Filtering, Rule-based Region Ranking.

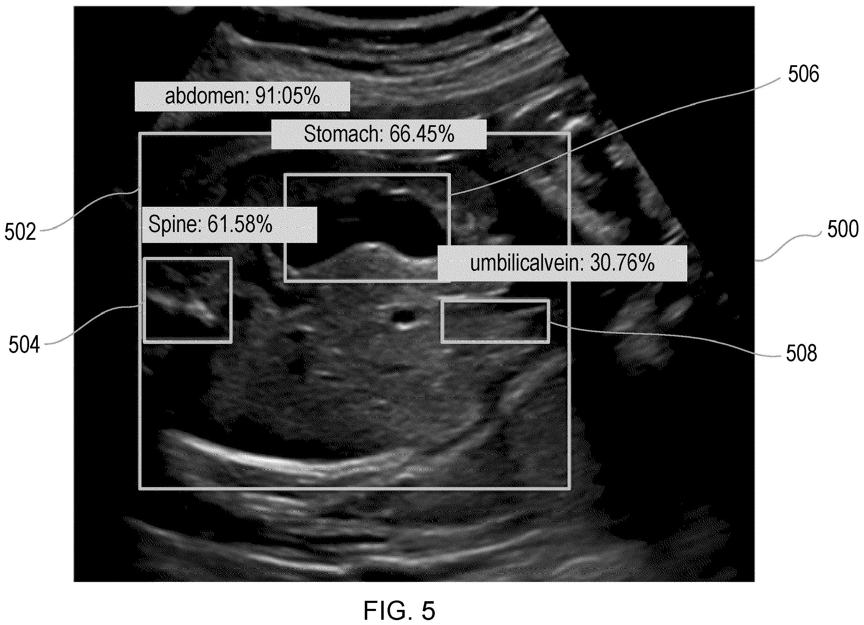

In one embodiment, a YOLO network is trained to detect anatomical structures which appear on prenatal ultrasound images. The output of the yolo network is bounding boxes of the detected structures and a confidence value for each detected structure, as is illustrated in Fig. 5. Each of the standardized anatomical views of the fetus is defined by a set of anatomical structures which either must be visible or must not be visible in the view. Some examples are given in Appendix I below.

In this embodiment, while the user is operating the ultrasound system in a 2D mode (from the user perspective), the central frame which is shown to the user is analyzed (see Fig.1 and 2b). The YOLO network is run on first, second and third 2D US images obtained (according to steps 302 and 204 described above) from first second and third scanning planes as illustrated in Fig. 4 above. Based on the confidence values of the detected individual anatomical structures, a combined score for the standardized views can be computed for each of the first, second and third 2D US images. The combined scores can be used to determine whether the target plane is closer to the first scanning plane, the second scanning plane or the third scanning plane.

In one example, the combined score could be a weighed sum of the "must have" and "must not have" anatomical structures associated with the target plane (the must not have structures with negative weights). In such an example, a higher score would indicate that a respective plane is closer to the target plane than a lower score.

Thus, in this manner, it can be determined whether the first scanning plane is closer to the target than on of the one or more other scanning planes associated with the one or more other 2D US images in the sub-volume surrounding the first scanning plane.

Turning back to the method 300, following step iii), the determination of whether the target is closer to the first 2D image (e.g. closer to the first scanning plane) or one of the one or more other 2D US images (or their associated scanning planes), the determination may be used in various different ways.

In some embodiments, in a fourth step, iv) the method 300 may further comprise providing directional guidance comprising a direction in which to move the US probe to image the target, based on the results of step iii).

For example, in embodiments, where a machine learning model to determine a first confidence score describing how closely the first 2D US image corresponds to the target and a second confidence score describing how closely the one or more other 2D US images correspond to the target, the directional guidance may indicate a direction toward a second scanning plane associated with one of the one or more other 2D US images, if the second confidence score is greater than the first confidence score (or vice versa).

Thus, confidence scores, as output from a machine learning model described above, may be used to identify the target (or target plane) that the user is currently aiming/searching for, as the 2D US image with the highest combined score. Confidence scores can also be used to provide directional guidance, as described above, for example they can be used to identify a view improvement direction in case one of the other 2D US images has a higher score for the target as compared to the first 2D US image (central view).

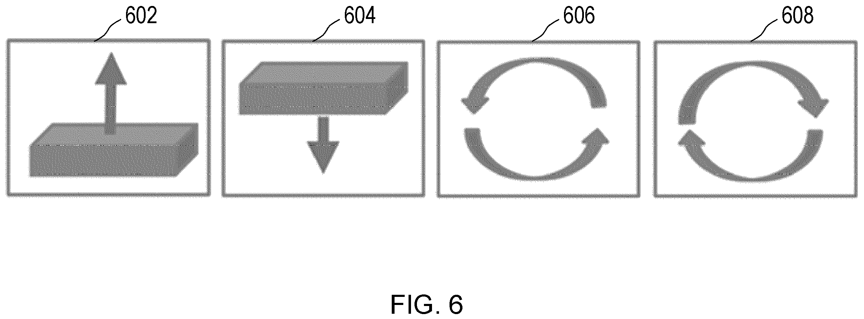

Directional guidance may be provided to the user in any manner, for example, as shown in Fig. 6, whereby the estimated direction of the improved view can be shown to the user by a set of icons displayed on the control screen, indicating shifts 602, 604, rotations 606, 608, and tilts.

Another option is a display directly on the transducer (small screen or LEDs).

As described above, the one or more other 2D US images may, in some embodiments, not be displayed to the sonographer, but rather used (solely) for the purposes of internal processing by an US imaging system to provide e.g. directional guidance to the sonographer. Thus, in some embodiments, the method 300 may comprise sending a message to cause a display to display only the first US image, the one or more other 2D US images being for use in determining the directional guidance.

For example, the US imaging system 200, the image acquisition process may alternate between steps i) and ii) e.g. it may alternate between obtaining a first 2D US image in the first scanning plane and obtaining one or more other 2D US images in a sub-volume around the first image plane. The first 2D US image in each iteration may be displayed to the user and the one or more other 2D US images may be used solely for the purpose of determining directional guidance.

The method 300 may further comprise repeating steps i)-iv) for consecutive groups of first 2D US image and one or more other 2D US images. The directional guidance may thus be updated in real time so as to provide real-time guidance as to the direction in which to move the US probe to image the target.

In another embodiment, the 2D US image determined to be closest (e.g. having the highest score) may be displayed to the user, saved or bookmarked as showing the target (or corresponding to the target plane). For example, if among the acquired 2D scanning planes around the center plane c (such as c-3,c-2,c-1, c, c+1, c+2, c+3), c-2 has the best score, then this closest image may be displayed to the user. This could be the case if one of the neighboring views provides a sufficiently good image (e.g. an image with a sufficiently high score).

It will further be appreciated that for an estimation of the improved view direction, the side views can be used not only to calculate combined sores, but also for multi-planar reformats which can be calculated based on several frames and which can indicate if, e.g., a tilt of the probe can lead to an increased combined score. For example, the method 300 may further comprising obtaining an oblique 2D US image at an oblique angle to the first scanning plane, by performing a multi-planar reformat of the first 2D US image and the one or more other 2D US images. In such embodiments, the directional guidance further comprises a tilt direction, the tilt direction having been determined based on the oblique angle and whether the target plane is closer to the first 2D US image or the oblique 2D US image.

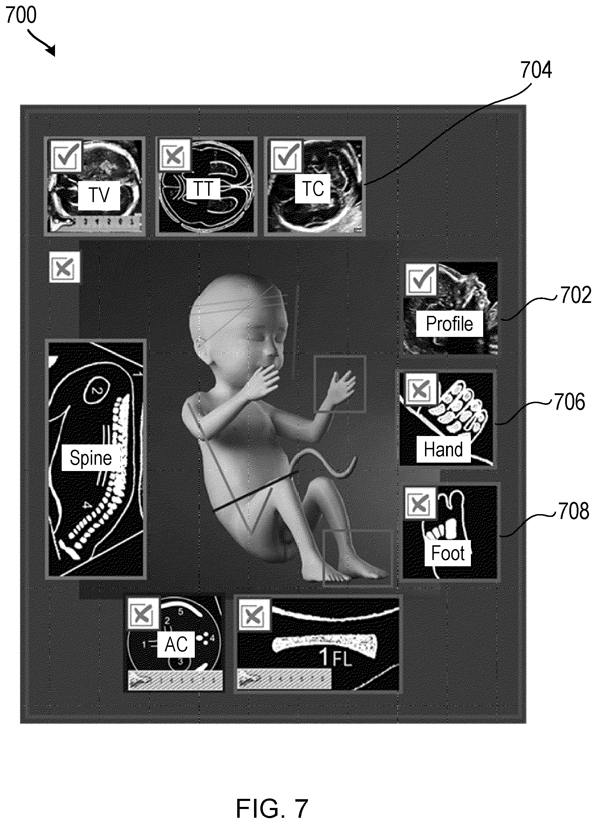

The embodiments described above may be combined with a workflow guidance system, supporting the user in following standardized screening protocols. This is illustrated in Fig. 7 which illustrates a Graphical User Interface (GUI 700) with a Visual workflow tracker for fetal screening, showing the coverage of target frames (e.g. that the profile 702 and TG 704 planes have been obtained, but the hand 706 and foot 708 profiles have not). In the above display, the view identified based on the combined score could be indicated.

Guiding the user beyond fetal screening, the system can be used for any clinical ultrasound application in which standardized view planes need to be acquired, defined by a set of anatomical structures.

Turning now to other embodiments, there is also provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### Appendix I

**Table 1**

| **Standardized view** | **Must have (Macro anatomy)** | **Must have (Micro anatomy)** | **Must not have** |
|---|---|---|---|
| Trans-thalamic brain plane | head | Thalamus or falx cerebri | Cerebellum, abdomen |
| Abdominal circumference plane | Abdomen | Spine, stomach, umbilical vein | Heart, femur, head |
| Femur biometry plane | | Femur | head, abdomen |

## Claims

1. A computer implemented method performed as part of an ultrasound, US, examination of a subject, the method comprising:
i) causing a three-dimensional, 3D, ultrasound probe to obtain a first 2D US image, wherein the first 2D US image is obtained in a first scanning plane of the probe;
ii) causing the US probe to obtain one or more other 2D US images in a sub-volume surrounding the first scanning plane, wherein the sub-volume has a smaller spanning angle than that of a 3D mode of the probe; and
iii) determining whether a target is closer to the first 2D US image or one of the one or more other 2D US images.

2. A method as in claim 1 further comprising:
iv) providing directional guidance comprising a direction in which to move the US probe to image the target, based on the results of step iii).

3. A method as in claim 2 further comprising:
sending a message to cause a display to display only the first 2D US image, the one or more other 2D US images being for use in determining the directional guidance.

4. A method as in claim 2 or 3 wherein step iii) comprises using a machine learning model to determine a first confidence score describing how closely the first 2D US image corresponds to the target and a second confidence score describing how closely the one or more other 2D US images correspond to the target; and
wherein the directional guidance indicates a direction toward a second scanning plane associated with the one or more other 2D US images, if the second confidence score is greater than the first confidence score.

5. A method as in claim 4 wherein the first confidence score is an aggregation of a plurality of confidence scores, each of the plurality of confidence scores reflecting a confidence with which the machine learning model detects a respective anatomical feature associated with the target, in the first 2D US image.

6. A method as in any one of claims 2 to 5, further comprising obtaining an oblique 2D US image at an oblique angle to the first scanning plane, by performing a multi-planar reformat of the first 2D US image and the one or more other 2D US images; and
wherein the directional guidance further comprises a tilt direction, the tilt direction having been determined based on the oblique angle and whether the target plane is closer to the first 2D US image or the oblique 2D US image.

7. A method as in any one of claims 2 to 6 further comprising:
repeating steps i)-iv) for consecutive groups of first 2D US image and one or more other 2D US images; and
updating the directional guidance in real time so as to provide real-time guidance as to the direction in which to move the US probe to image the target.

8. A method as in any one of the preceding claims wherein the target is a target structure in the subject, or a target plane through the subject.

9. A method as in any one of the preceding claims wherein the one or more other 2D US images are obtained by means of a wobble about the central scanning plane of the probe.

10. A method as in any one of the preceding claims wherein the one or more other 2D US images are in scanning planes adjacent to the first scanning plane.

11. An apparatus for performing an ultrasound, US, examination of a subject, the apparatus comprising:
a memory comprising instruction data representing a set of instructions; and
a processor configured to communicate with the memory and to execute the set of instructions, wherein the set of instructions, when executed by the processor, cause the processor to:
i) obtain a first 2D US image using a three-dimensional, 3D, ultrasound imaging probe, wherein the first 2D US image is obtained in a first scanning plane of the probe;
ii) obtain one or more other 2D US images in a sub-volume surrounding the first scanning plane, wherein the sub-volume has a smaller spanning angle than that of a 3D mode of the probe; and
iii) determine whether a target is closer to the first 2D US image or one of the one or more other 2D US images.

12. An ultrasound, US, imaging system comprising a three-dimensional, 3D, ultrasound probe, wherein the US imaging system is configured to cause the probe to operate in a scanning mode whereby the probe:
v) obtains a first 2D US image in a first scanning plane of the probe;
vi) obtains one or more other 2D US images in a sub-volume surrounding the first scanning plane, wherein the sub-volume has a smaller spanning angle than that of a 3D mode of the probe; and
repeats steps v) and vi) in a continuous manner.

13. The ultrasound imaging system of claim 12 further comprising the apparatus of claim 12 and a display to display the first 2D US image and directional guidance, the directional guidance comprising a direction in which to move the US probe to image the target, based on the results of step iii); and
wherein only the first 2D image is displayed on the display, the one or more other 2D US images being for use in determining the directional guidance.

14. The ultrasound system of claim 12 or 13 wherein the ultrasound system is configured to repeat steps i), ii), and iii), displaying only the first image to the user in each iteration.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 10.
